# EUROPÄISCHE PATENTSCHRIFT

(11) **EP 3 299 832 B1**
(45) Veröffentlichungstag und Bekanntmachung des Hinweises auf die Patenterteilung: **19.12.2018**
(21) Anmeldenummer: 16190403.2
(22) Anmeldetag: 23.09.2016
(51) Int. Cl.: G01R 33/36, H01R 12/91

(54) **LOKALSPULE MIT EINEM ERSTEN VERBINDUNGSTEIL EINER STECKVERBINDUNG, UND MAGNETRESONANZGERÄT ODER PATIENTENLIEGE MIT EINEM ZUGEHÖRIGEN ZWEITEN VERBINDUNGSTEIL**
LOCAL COIL WITH A FIRST CONNECTING ELEMENT OF A CONNECTOR, AND MAGNETIC RESONANCE APPARATUS OR PATIENT TABLE WITH A CORRESPONDING SECOND CONNECTING ELEMENT
BOBINE LOCALE AVEC UN PREMIER ÉLÉMENT DE RACCORDEMENT D'UN CONNECTEUR, ET APPAREIL À RÉSONANCE MAGNÉTIQUE OU LIT D'EXAMEN AVEC UN DEUXIÈME ÉLÉMENT DE RACCORDEMENT CORRESPONDANT

(43) Veröffentlichungstag der Anmeldung: 28.03.2018
(73) Patentinhaber: Siemens Healthcare GmbH, 91052 Erlangen (DE)
(72) Erfinder: Kraus, Wolfgang, 90403 Nürnberg (DE); Kundner, Thomas, 91054 Buckenhof (DE)

(56) Entgegenhaltungen:
- WO-A1-2015/196210
- CN-Y- 2 559 133
- FR-A1- 2 873 237
- US-A- 6 062 886

## Beschreibung

Die Erfindung betrifft eine Lokalspule mit einem ersten Verbindungsteil, und ein Magnetresonanzgerät oder eine Patientenliege mit einem zweiten Verbindungsteil, wobei das erste und das zweite Verbindungsteil ausgebildet sind, eine Steckverbindung zum Einsatz in einem Magnetresonanzgerät einzugehen.

In der Medizintechnik zeichnet sich die Bildgebung mittels Magnetresonanz (MR), auch Magnetresonanztomographie (MRT, engl. Magnetic Resonance Imaging, MRI) genannt, durch hohe und variable Weichteilkontraste aus. Hierbei werden mit Hilfe eines Magnetresonanzgeräts Anregungspulse in einen Patienten eingestrahlt, welche im Patienten Magnetresonanzsignale auslösen. Die Magnetresonanzsignale werden durch elektrisch leitende Schleifen, sogenannten Spulen und/oder Antennen, empfangen. Dabei wird durch das Magnetresonanzsignal eine Spannung in der Spule induziert. Die induzierte Spannung wird üblicherweise mittels eines rauscharmen Vorverstärkers verstärkt an eine Empfangselektronik des Magnetresonanzgeräts weitergeleitet.

Die Empfangsspulen werden idealerweise möglichst nahe an dem Patienten angeordnet. Deshalb werden diese auch als Lokalspulen (engl. local coils) bezeichnet. Üblicherweise sind die Lokalspulen nicht fest mit dem Magnetresonanzgerät verbunden, sondern werden über eine Steckverbindung an das Magnetresonanzgerät angeschlossen.

Konventionelle Steckverbindungen umfassen beispielsweise spulenseitig ein erstes Verbindungsteil, beispielsweise einen Stecker an einem Kabel der Lokalspule, und geräteseitig ein zweites Verbindungsteil, beispielsweise eine Buchse an einer Patientenliege der Magnetresonanzanlage.

Der spulenseitige Stecker umfasst oftmals Kontaktelemente, insbesondere nach außen weisende Kontaktstifte, die freiberührbar sind. Damit besteht die Gefahr einer mechanischen Beschädigung, etwa durch Verbiegen der Kontaktstifte.

Die zum Stecker korrespondierende Buchse umfasst üblicherweise Kontaktelemente, beispielsweise nach innen weisende Kontaktöffnungen. Die Kontaktöffnungen sind oftmals in einem Isolierkörper angeordnet, insbesondere eingepresst. Ferner kann Buchse eine Abdeckung umfassen, beispielsweise mit einer gefederten Schiebemechanik. Die Abdeckung kann insbesondere vor direkter Berührung der Kontaktelemente und/oder vor Flüssigkeitseintritt schützen. Insbesondere sind Kontaktöffnungen in der Buchse oftmals zurückversetzt angeordnet, um den Berührschutz zusätzlich zu erhöhen.

Insbesondere bei einer geöffneten Abdeckung können trotzdem Flüssigkeiten in den Isolierkörper eintreten und beispielsweise zu einer Korrosion einer sich möglicherweise anschließenden Leiterplatte führen. Außerdem können nach einer Reinigung in der Buchse Flüssigkeitsrückstände von Reinigungsmitteln verbleiben, die eine Materialversprödung verursachen können, da die Reinigungsmittel in Vertiefungen der Buchse sich sammeln und über lange Zeit einwirken können.

Die Druckschrift FR 2 873 237 A1 offenbart eine flüssigkeitsdichte Steckverbindung mit zwei Verbindungseinheiten. Die Verbindungseinheiten umfassen eine bewegliche Schutzabdeckung mit elektrisch leitfähigen Einsätzen. Ferner umfassen die Verbindungseinheiten im Gehäuseinneren angeordnete, ortsfeste Kontakte. Beim Verbinden der Verbindungseinheiten werden Schutzabdeckungen von außen nach innen bewegt, um die elektrisch leitfähigen Einsätze der Schutzabdeckungen mit den ortsfesten Kontakten zu kontaktieren.

Die Druckschrift US 6062886 offenbart eine ähnliche wasserdichte Steckverbindung, bei der eine Buchse innen abgeordnete, ortsfeste Klemmen und eine elastische Abdeckung mit leitfähigen hohlen Schäften aufweist. Beim Verbinden der Steckverbindung wird ein Stecker in die Schäfte eingeführt und die elastische Abdeckung nach ins Innere der Buchse gedrückt, um die Schäfte in die Klemmen zu schieben.

Die Druckschrift CN 2 559 133 Y offenbart einen Wasserkocher mit einer drehbaren elektrischen Verbindung. Beim Verbinden der elektrischen Verbindung wird ein Hebel mit einem elektrischen Kontakt in Rotation versetzt, so dass der elektrische Kontakt einen anderen elektrischen Kontakt berührt.

Die Druckschrift WO 2015/196210 A1 offenbart eine Steckverbindung mit Kontakten, die durch eine bewegliche Platte geschützt werden.

Der Erfindung liegt die Aufgabe zugrunde, ein Magnetresonanzgerät oder eine Patientenliege mit einem zweiten Verbindungsteil, sowie eine Lokalspule mit einem ersten Verbindungsteil anzugeben, wobei das erste und das zweite Verbindungsteil dazu ausgebildet sind, eine MR-kompatible Steckverbindung einzugehen, wobei die Steckverbindung leicht zu reinigen ist.

Die Aufgabe wird durch die Merkmale der unabhängigen Ansprüche gelöst. Vorteilhafte Ausgestaltungen sind in den Unteransprüchen beschrieben.

Demnach wird eine Lokalspule mit einem ersten Verbindungsteil, und ein Magnetresonanzgerät oder eine Patientenliege mit einem zweiten Verbindungsteil vorgeschlagen, wobei das erste und das zweite Verbindungsteil ausgebildet sind, eine Steckverbindung zum Einsatz in einem Magnetresonanzgerät einzugehen. Dabei sind das erste und das zweite Verbindungsteil ausgebildet, lösbar miteinander verbunden zu werden. Das erste Verbindungsteil umfasst eine erste Kontaktfläche und das zweite Verbindungsteil umfasst eine erste Kontaktplatte, eine zweite Kontaktplatte und ein Gehäuse. Dabei ist die zweite Kontaktplatte relativ zum Gehäuse beweglich angeordnet. In einem verbundenen Zustand ist die erste Kontaktplatte zwischen der ersten Kontaktfläche und der zweiten Kontaktplatte angeordnet. Zudem umfasst die elektrische Steckverbindung eine mechanische Hebevorrichtung, die ausgebildet ist, bei einem Verbinden des ersten Verbindungsteils mit dem zweiten Verbindungsteil die zweite Kontaktplatte, insbesondere relativ zum Gehäuse, in Richtung der ersten Kontaktplatte zu bewegen.

Das erste Verbindungsteil kann beispielsweise von einem Handstecker umfasst werden. Das zweite Verbindungsteil kann beispielsweise von einem Steckerfeld, insbesondere der Patientenliege, umfasst werden. Das erste Verbindungsteil und das zweite Verbindungsteil können zur Herstellung einer Verbindung miteinander in Kontakt gebracht werden und/oder zu Trennung einer Verbindung voneinander entfernt werden. Der Steckverbinder ist also vorzugsweise ausgebildet, Leitungen zu trennen und/oder zu verbinden, über welche beispielsweise Magnetresonanzsignale übertragen werden können. Da die Steckverbindung zum Einsatz in einem Magnetresonanzgerät vorgesehen ist, umfasst die Steckverbindung vorzugsweise ausschließlich MR-kompatible, insbesondere nichtmagnetische, Materialien.

Die Steckverbindung ist eine elektrische Steckverbindung, d. h. im verbundenen Zustand wird zumindest ein elektrischer Kontakt zwischen dem ersten Verbindungsteil und dem zweiten Verbindungsteil hergestellt. Über den zumindest einen Kontakt können elektrische Signale und/oder elektrische Energie übertragen werden.

Bevorzugt ist die erste Kontaktplatte außenseitig am zweiten Verbindungsteil angeordnet, während die zweite Kontaktplatte innenseitig im Gehäuse angeordnet ist. Vorzugsweise erfolgt beim Verbinden die Bewegung der zweiten Kontaktplatte von innen nach außen, d. h. die bewegliche zweite Kontaktplatte ist einem unverbundenen Zustand innen in einem gewissen Abstand zur ersten Kontaktplatte angeordnet und wird zur Herstellung einer Verbindung nach außen bewegt, um sie mit der ersten Kontaktplatte zu kontaktieren. Im unverbundenen Zustand sind die erste Kontaktplatte und die zweite Kontaktplatte also galvanisch getrennt. Das zweite Verbindungsteil kann durch die Bewegung der innen liegenden zweiten Kontaktplatte, insbesondere zur Aufhebung der galvanischen Trennung, nach außen hin starr ausgestaltet sein, d. h. das zweite Verbindungsteil weist keine flexible und/oder bewegliche Oberfläche auf. Dadurch kann das zweite Verbindungsteil, insbesondere die Oberfläche des zweiten Verbindungsteils, leichter gereinigt werden.

Zur beweglichen Anordnung der zweiten Kontaktplatte im Gehäuse kann das Gehäuse insbesondere eine Führungseinheit umfassen, die beispielsweise eine Linearführung und/oder einen Schacht aufweist, in der die zweite Kontaktplatte beweglich gelagert ist.

Vorzugsweise umfasst die erste Kontaktplatte eine äußere Fläche und eine innere Fläche, die sich beispielsweise wie bei einer Leiterplatte gegenüber liegen. Im verbundenen Zustand ist vorzugsweise die äußere Fläche der ersten Kontaktplatte der ersten Kontaktfläche des ersten Verbindungsteils zugewandt. Insbesondere berührt im verbunden Zustand die äußere Fläche der ersten Kontaktplatte die Kontaktfläche des ersten Verbindungsteils. Ferner berührt im verbunden Zustand vorzugsweise die innere Fläche der ersten Kontaktplatte die zweite Kontaktplatte. Zudem umfasst die erste Kontaktplatte vorzugsweise zumindest eine Durchkontaktierung, über welche die Kontaktfläche des ersten Verbindungsteils mit der zweiten Kontaktplatte im verbundenen Zustand kontaktierbar ist.

Möglicherweise weist auch das Gehäuse eine äußere Fläche auf, die im verbundenen Zustand der ersten Kontaktfläche des ersten Verbindungsteils zugewandt ist. Die äußere Fläche des zweiten Verbindungsteils, insbesondere der ersten Kontaktplatte und/oder möglicherweise des Gehäuses, weisen vorteilhafterweise eine glatte und/oder in sich geschlossene Oberfläche auf, so dass diese leicht gereinigt werden kann. Eine glatte Oberfläche kann hier als eine Oberfläche angesehen werden, deren maximale Erhöhungen kleiner als 5 mm, vorzugsweise kleiner als 1 mm, sind. Insbesondere kann die erste Kontaktplatte zumindest ein Kontaktelement und einen Isolierkörper umfassen, in dem das zumindest eine Kontaktelement angeordnet ist, wobei das zumindest eine Kontaktelement auf der äußeren Fläche bündig zu dem Isolierkörper angeordnet ist.

Die erste Kontaktplatte ist vorzugsweise ortsfest zum Gehäuse des zweiten Verbindungsteils angeordnet, d. h. das Gehäuse ist vorzugsweise starr mit der ersten Kontaktplatte verbunden. Die erste Kontaktplatte kann auch zumindest teilweise monolithisch mit dem Gehäuse des zweiten Verbindungsteils verbunden sein, d. h. zumindest ein Teil der ersten Kontaktplatte, insbesondere der Isolierkörper, und das Gehäuse sind einstückig ausgebildet. Die Kontaktplatte kann beispielsweise in das Gehäuse eingespritzt sein und/oder mit Dichtungen an dem Gehäuse befestigt sein. Das Gehäuse kann beispielsweise zumindest teilweise aus Kunststoff bestehen.

Insbesondere findet durch die ortsfeste Anordnung keine Relativbewegung zwischen der ersten Kontaktplatte und dem Gehäuse statt. Das schließt jedoch nicht aus, dass die erste Kontaktplatte, insbesondere innenseitige und/oder ins Gehäuseinnere weisende, Elemente umfasst, die relativ zur ersten Kontaktplatte beweglich, insbesondere gefedert, angeordnet sind.

Wie bereits beschrieben, wird durch die bewegliche Anordnung der zweiten Kontaktplatte ermöglicht, dass in einem unverbundenen Zustand die zweite Kontaktplatte zur ersten Kontaktplatte beabstandet ist. Somit kann eine galvanische Trennung zwischen der ersten und zweiten Kontaktplatte erreicht werden. Der bevorzugte Abstand kann gemäß Anforderungen an Luft- und Kriechstrecken ermittelt werden. Durch die galvanische Trennung kann die Sicherheit bei der Handhabung der Steckverbindung erhöht werden, insbesondere dann, wenn die zweite Kontaktplatte mit elektrischer Spannung beaufschlagt ist. Es ist also keine weitere Abdeckung notwendig, um insbesondere den Berührschutz gegenüber einem Patienten zu gewährleisten.

Der Abstand zwischen der zweite Kontaktplatte und der ersten Kontaktplatte ist vorzugsweise durch Bewegung der zweiten Kontaktplatte relativ zum Gehäuse veränderbar. Die mechanische Hebevorrichtung bewirkt vorzugsweise, dass beim Verbinden des ersten Verbindungsteils mit dem zweiten Verbindungsteil, also bei einer Änderung von einem unverbundenen Zustand in einen verbundenen Zustand, der Abstand zwischen der zweite Kontaktplatte und der ersten Kontaktplatte reduziert wird. Der Abstand wird beim Verbinden vorzugsweise soweit reduziert, dass sich im verbundenen Zustand die erste und die zweite Kontaktplatte berühren. Somit kann die galvanische Trennung der zwischen der ersten und zweiten Kontaktplatte aufgehoben werden.

Vorzugsweise bewegt sich beim Verbinden des ersten Verbindungsteils mit dem zweiten Verbindungsteil das erste Verbindungsteil, insbesondere relativ zum Gehäuse des zweiten Verbindungsteils, in eine erste Verbindungsrichtung und die zweite Kontaktplatte der zweiten Verbindungsrichtung in eine zweite Verbindungsrichtung, insbesondere relativ zum Gehäuse des zweiten Verbindungsteils, wobei die erste Verbindungsrichtung und die zweite Verbindungsrichtung einen Winkel von mindestens 90°, insbesondere mindestens 120°, und höchstens 180° einschließen. Insbesondere sind die erste Verbindungsrichtung und die zweite Verbindungsrichtung zueinander entgegengesetzt, d.h. sie schließen einen Winkel von 180° ein.

Somit kann unmittelbar beim Verbinden des ersten Verbindungsteils mit dem zweiten Verbindungsteil eine galvanische Trennung zwischen der ersten und der zweiten Kontaktplatte aufgehoben werden.

Ferner wird vorgeschlagen, dass die Hebevorrichtung ausgebildet ist, bei dem Verbinden des ersten Verbindungsteils mit dem zweiten Verbindungsteil eine mechanische Kraft vom ersten Verbindungsteil auf die zweite Kontaktplatte zu übertragen. Insbesondere setzt die Hebevorrichtung keine magnetische Kraft ein, da sich diese nicht für den Einsatz in einem Magnetresonanzgerät eignen würde.

Vorzugsweise wird die mechanische Kraft ohne Zuhilfenahme der ersten Kontaktplatte übertragen, d. h. der Kraftfluss erfolgt nicht über die erste Kontaktplatte. Durch die mechanische Kraft kann zweite Kontaktplatte in Richtung der ersten Kontaktplatte bewegt werden.

Eine Ausführungsform sieht vor, dass die die Hebevorrichtung ein erstes Hebevorrichtungsteil und ein zweites Hebevorrichtungsteil umfasst, wobei das erste Verbindungsteil das erste Hebevorrichtungsteil umfasst und das zweite Verbindungsteil das zweite Hebevorrichtungsteil umfasst. Dabei wird bei dem Verbinden des ersten Verbindungsteils mit dem zweiten Verbindungsteil durch das erste Hebevorrichtungsteil eine erste mechanische Kraft auf das zweite Hebevorrichtungsteil eingeleitet, so dass durch das zweite Hebevorrichtungsteil eine zweite mechanische Kraft auf die zweite Kontaktplatte eingeleitet wird. Dadurch kann die zweite Kontaktplatte in Richtung der ersten Kontaktplatte bewegt werden.

Der Kraftfluss erfolgt also vorzugsweise vom ersten Hebevorrichtungsteil über das zweite Hebevorrichtungsteil auf die zweite Kontaktplatte. Die Hebevorrichtung ist vorteilhafterweise so konstruiert, dass das erste Hebevorrichtungsteil, das zweite Hebevorrichtungsteil und die Kontaktplatte derart wechselwirken, dass beim Verbinden des ersten Verbindungsteils mit dem zweiten Verbindungsteil die zweite Kontaktplatte in Richtung der ersten Kontaktplatte bewegt wird.

Vorzugsweise ist die erste mechanische Kraft verschieden zur zweiten mechanischen Kraft ausgerichtet, d. h. es erfolgt durch die Hebevorrichtung eine Umlenkung der die Bewegung der zweiten Kontaktplatte auslösenden ersten mechanischen Kraft.

Vorzugsweise ist zumindest eine Komponente der ersten mechanische Kraft parallel zur ersten Verbindungsrichtung ausgerichtet und/oder die zumindest eine Komponente der zweiten mechanischen Kraft parallel zur zweiten Verbindungsrichtung ausgerichtet.

Vorzugsweise weist das Gehäuse des zweiten Verbindungsteils eine Aussparung auf, wobei das erste Hebevorrichtungsteil zumindest ein vorstehendes Element umfasst, das in der zumindest einen Aussparung des Gehäuses anordbar ist. Bevorzugt ist die zumindest eine Aussparung neben der ersten Kontaktplatte angeordnet.

Die zumindest eine Aussparung weist vorzugsweise eine längliche, insbesondere zylinderförmige, Geometrie auf. Die längliche Geometrie weist vorzugsweise eine Längsachse auf. Die Längsachse ist bevorzugt parallel zur ersten Verbindungsrichtung. Vorzugsweise bewirkt die Geometrie der zumindest einen Aussparung eine Zwangsführung des ersten Verbindungsteils beim Verbinden mit dem zweiten Verbindungsteil.

Die erste Kontaktplatte weist vorzugsweise eine Hauptausdehnungsfläche auf. Die Längsachse der Geometrie der Aussparung schließt mit der Normalen der Hauptausdehnungsfläche der ersten Kontaktplatte vorzugsweise einen Winkel von höchstens 90° ein. Insbesondere schließt die Längsachse der Geometrie der Aussparung mit der Normalen der Hauptausdehnungsfläche der ersten Kontaktplatte einen Winkel von im Wesentlichen 0° ein, d.h. die Längsachse ist im Wesentlichen senkrecht zur Hauptausdehnungsfläche der ersten Kontaktplatte orientiert.

Die zumindest eine Aussparung weist vorzugsweise zumindest eine Aussparungsöffnung auf, durch die das zumindest eine vorstehende Element, beispielsweise ein zylinderförmiger Stift, sich in die zumindest eine Aussparung schieben lässt. Bevorzugt ist in und/oder an der zumindest einen Aussparung zumindest ein Teil des zweiten Hebevorrichtungsteils angeordnet. Beim Hineinschieben des zumindest eine vorstehenden Elements in die zumindest eine Aussparung kann durch das vorstehende Element vorteilhafterweise eine mechanische Kraft, insbesondere die erste mechanische Kraft, auf das zweite Hebevorrichtungsteil eingeleitet werden.

Vorzugsweise umfasst das zweite Verbindungsteil zumindest eine Aussparungsabdeckung, um die zumindest eine Aussparungsöffnung der zumindest einen Aussparung in einem unverbundenen Zustand abzudecken. So können Flüssigkeiten und/oder Schmutz in die zumindest eine Aussparung nicht oder in geringerem Maße eintreten. Vorteilhafterweise ist die beweglich angeordnet, insbesondere wegklappbar, so dass das zumindest eine vorstehende Element in die zumindest eine Aussparung beim Verbinden des ersten Verbindungsteils mit dem zweiten Verbindungsteil in die zumindest eine Aussparung eindringen kann.

Die zumindest eine Aussparungsabdeckung ist vorzugsweise ausgebildet, dass sie im unverbundenen Zustand bündig mit der sie umgebenden Oberfläche abschließt und erst beim Verbinden geöffnet wird.

Vorzugsweise umfasst das zweite Verbindungsteil eine Ableitungseinheit, die ausgebildet ist, etwaige Fremdsubstanzen, insbesondere Flüssigkeiten und/oder Schmutz, aus der zumindest einen Aussparung abzuleiten. Die Ableitungseinheit kann beispielsweise einen oder mehrere nach außen führende Ableitungskanäle umfassen. Die Ableitungseinheit ist vorzugsweise an einer zur Aussparungsöffnung beabstandeten, insbesondere gegenüber liegenden, Seite der zumindest einen Aussparung angeordnet. Insbesondere können die etwaigen Fremdsubstanzen durch die Schwerkraft nach unten abgeleitet werden, wenn das zweite Verbindungsteil so ausgerichtet ist, dass die zumindest eine Aussparungsöffnung nach oben zeigt.

Insbesondere kann die zumindest eine Aussparung in einem separaten Bereich des Gehäuses eingebracht sein, der nach unten geöffnet ist, so dass eindringende Flüssigkeiten direkt abgeleitet werden können. Diese Bereiche können derartig ausgestaltet werden, dass eine leichte Reinigung mit üblichen Hilfsmitteln, z. B. Wattestäbchen, möglich ist.

Vorteilhafterweise ist zweites Verbindungsteil derart ausgestaltet, dass keine Fremdsubstanzen von der zumindest einen Aussparung zur ersten Kontaktplatte gelangen können. Damit kann verhindert werden, dass die Fremdsubstanzen mit etwaigen elektrisch aktiven Komponenten des zweiten Verbindungsteils in Kontakt kommen und beispielsweise einen Kurzschluss verursachen.

Bevorzugt umfasst das zweite Hebevorrichtungsteil zumindest eine Wippe und/oder zumindest einen Hebekeil. Diese Elemente eigenen sich besonders, um eine mechanische Kraft, insbesondere die erste mechanische Kraft, vom ersten Verbindungsteil auf die zweite Kontaktplatte zu übertragen. Insbesondere ermöglichen diese Elemente eine Umlenkung der mechanischen Kraft, d. h. einen nicht-geradlinigen Kraftfluss. Durch einen nicht-geradlinigen Kraftfluss kann beim Verbinden des Steckverbinders die zweite Kontaktplatte in eine Richtung, insbesondere in die zweite Verbindungsrichtung, bewegt werden, die sich von der Bewegungsrichtung des ersten Verbindungsteils, insbesondere der ersten Verbindungsrichtung, unterscheidet.

Eine Ausführungsform der Erfindung sieht vor, dass die zumindest eine Wippe eine erste Wippenfläche und eine zweite Wippenfläche aufweist, wobei bei einer Einleitung der mechanischen Kraft, insbesondere der ersten mechanischen Kraft, in die erste Wippenfläche die zweite Wippenfläche gedreht wird. Durch die Drehung kann eine mechanische Kraft, insbesondere die zweite mechanische Kraft, in die zweite Kontaktplatte eingeleitet werden. Durch eine Einleitung einer mechanischen Kraft in einen Gegenstand wirkt die mechanische Kraft auf den Gegenstand ein, d. h. die mechanische Kraft greift an dem Gegenstand an.

Vorzugsweise ist die Wippe drehbar im Gehäuse gelagert, um die Drehung der Wippe zu ermöglichen. Vorzugsweise ist erste Wippenfläche und die zweite Wippenfläche starr miteinander verbunden, insbesondere einstückig ausgebildet. Dadurch kann besonders effektiv die mechanische Kraft von der ersten Wippenfläche auf die zweite Wippenfläche übertragen werden.

Eine weitere Ausführungsform der Erfindung sieht vor, dass der zumindest eine Hebekeil eine erste Keilfläche aufweist, wobei der zumindest eine Hebekeil derart angeordnet und/oder ausgebildet ist, dass das erste Hebevorrichtungsteil beim Verbinden des ersten Verbindungsteils mit dem zweiten Verbindungsteil in eine Richtung, insbesondere in die erste Verbindungsrichtung, bewegt wird, die mit der Normalen der ersten Keilfläche einen ersten Neigungswinkel größer als 0°, insbesondere größer als 20°, und kleiner als 90°, insbesondere kleiner als 70°, einschließt.

Dadurch kann beim Verbinden des ersten Verbindungsteils mit dem zweiten Verbindungsteil der zumindest eine Hebekeil bei einer Einleitung einer mechanischen Kraft, insbesondere der ersten mechanischen Kraft, auf die erste Keilfläche in eine zur Bewegungsrichtung des ersten Hebevorrichtungsteils verschiedenen Verschiebungsrichtung bewegt werden. Die mechanische Kraft kann insbesondere durch das vorstehende Element in die erste Keilfläche eingeleitet werden.

Vorteilhafterweise wirkt die erste Keilfläche wie eine schiefe Ebene, die eine mechanische Kraft umlenkt. Insbesondere kann die erste mechanische Kraft durch die schiefe Ebene in mehrere Anteile zerlegt werden, wobei einer der Anteile parallel zur Verschiebungsrichtung ausgerichtet ist.

Der erste Neigungswinkel ist vorzugsweise so ausgelegt, dass bei einer Einleitung einer mechanischen Kraft ein mögliches Verkanten des zumindest einen Hebekeils nicht auftritt. Ferner ist der erste Neigungswinkel ist vorzugsweise so ausgelegt, dass etwaige Reibungskräfte überwunden werden, die bei ungünstiger Wahl des ersten Neigungswinkels eine Bewegung des zumindest einen Hebekeils verhindern würden.

Vorzugsweise weist der zumindest eine Hebekeil und eine zweite Keilfläche auf, deren Normale gegenüber der Verschiebungsrichtung einen zweiten Neigungswinkel größer als 0°, insbesondere größer als 20°, und kleiner als 90°, insbesondere kleiner als 70°, einschließt.

Dadurch kann durch die zweite Keilfläche in die zweite Kontaktplatte eine mechanische Kraft, insbesondere die zweite mechanische Kraft, eingeleitet werden, wodurch die zweite Kontaktplatte in eine zur Verschiebungsrichtung verschiedenen Richtung, insbesondere in die zweite Verbindungsrichtung, bewegt werden kann. Dies erfolgt vorzugsweise gemäß dem vorab beschriebenen Prinzip einer Kraftaufspaltung mit Hilfe einer schiefen Ebene. Dabei ist die zweite Keilfläche vorzugsweise gegenläufig zur ersten Keilfläche.

Auch der zweite Neigungswinkel ist vorzugsweise so ausgelegt, dass bei einer Einleitung einer mechanischen Kraft ein mögliches Verkanten des zumindest einen Hebekeils nicht auftritt, und/oder dass etwaige Reibungskräfte überwunden werden, die bei ungünstiger Wahl des zweiten Neigungswinkels eine Bewegung des zumindest einen Hebekeils verhindern würden.

Vorzugsweise ist erste Keilfläche und die zweite Keilfläche starr miteinander verbunden, insbesondere einstückig ausgebildet. Dadurch kann besonders effektiv die mechanische Kraft von der ersten Keilfläche auf die zweite Keilfläche übertragen werden.

Insbesondere kann die Hebevorrichtung ausgestaltet sein, dass die erste Verbindungsrichtung senkrecht zur Verschiebungsrichtung und/oder die zweite Verbindungsrichtung senkrecht zur Verschiebungsrichtung sind. Somit ergäbe ich ein rechtwinkliger Kraftfluss vom ersten Verbindungsteil, insbesondere dem etwaigen zumindest einem vorstehenden Element, auf die zweite Kontaktplatte.

Bevorzugt umfasst das zweite Verbindungsteil eine Federeinheit, die zwischen der zweiten Kontaktplatte und der ersten Kontaktplatte eine abstoßende Kraft ausübt. Somit kann sichergestellt werden, dass die erste Kontaktplatte zur zweiten Kontaktplatte im unverbundenen Zustand beabstandet, insbesondere galvanisch getrennt, ist.

Beim Verbinden der Steckverbindung wird vorzugsweise gegen den Federdruck der Federeinheit die zweite Kontaktplatte bewegt und/oder gegen die innere Fläche der ersten Kontaktplatte gepresst. Bei einem Lösen der Steckverbindung wird vorteilhafterweise die zweite Kontaktplatte, beispielsweise über die Federeinheit wieder in von der ersten Kontaktplatte wegbewegt und somit insbesondere automatisch eine galvanische Trennung vorgenommen.

Vorzugsweise umfasst die Federeinheit zumindest eine Druckfeder. Druckfedern sind gut verfügbar und lassen sich besonders gut in die zweite Verbindungsteil integrieren.

Vorzugsweise umfasst die Steckverbindung eine Einrastvorrichtung, damit im verbundenen Zustand eine dauerhafte Kontaktierung, insbesondere gegen den etwaigen Gegendruck durch die Federeinheit, sichergestellt werden kann.

Die erste Kontaktfläche weist mindest ein erstes elektrisches Kontaktelement auf, die erste Kontaktplatte weist zumindest ein zweites elektrisches Kontaktelement auf und die zweite Kontaktplatte weist zumindest ein drittes elektrisches Kontaktelement auf. Dabei berührt in dem verbundenen Zustand der zumindest eine erste elektrischen Kontakt den zumindest einen zweiten elektrischen Kontakt und der zumindest eine zweite elektrische Kontakt den zumindest einen dritten elektrischen Kontakt.

Somit kann eine elektrische Verbindung zwischen dem ersten Steckverbindungsteil und dem zweiten Steckverbindungsteil hergestellt werden.

Vorzugsweise sind die Kontaktelemente unmagnetisch, um keine Störungen bei einer Magnetresonanzmessung zu verursachen.

Vorzugsweise weisen das zumindest eine erste elektrische Kontaktelement und/oder das zumindest eine zweite elektrische Kontaktelement und/oder das zumindest eine dritte elektrische Kontaktelement zumindest einen Federkontakt, insbesondere zumindest einen Federstiftkontakt und/oder zumindest einen Stanzbiegekontakt, auf.

Vorteilhafterweise weist das zumindest eine zweite elektrische Kontaktelement nur auf der inneren Fläche der ersten Kontaktplatte einen Federkontakt auf. Auf der äußeren Fläche könnten Federkontakte eine Reinigung des zweiten Verbindungsteils erschweren. Ferner werden insbesondere für die erste Kontaktfläche und die zweite Kontaktplatte Federkontakte vorgeschlagen, die jeweils auf die erste Kontaktplatte angefedert werden können. Insbesondere können die Federkontakte der ersten Kontaktfläche auf eine Seite und/oder die Federkontakte der zweiten Kontaktplatte auf eine andere Seite der ersten Kontaktplatte angefedert werden. So kann beispielsweise beim Verbinden zumindest ein gefedertes erstes und/oder drittes Kontaktelement auf zumindest ein ungefedertes zweites Kontaktelement gepresst werden.

Durch die Verwendung von Federkontakten ist kein konventionelles "Einstecken" wie bei klassischen Pin-Buchsen-Kontakten notwendig. Mit Hilfe von gefederten Kontaktelementen können sichere Kontaktierungen erzeugt und Fertigungstoleranzen ausgeglichen werden.

Bevorzugt weist die erste Kontaktfläche mehrere erste elektrische Kontakte auf, die in einem ersten Muster angeordnet sind, und die erste Kontaktplatte mehrere zweite elektrische Kontakte auf, die in einem zweiten Muster angeordnet sind, und die zweite Kontaktplatte mehrere dritte elektrische Kontakte aufweist, die in einem dritten Muster angeordnet sind. Dabei sind das erste Muster, das zweite Muster und das dritte Muster zumindest teilweise deckungsgleich.

Vorzugsweise sind die Kontaktelemente elektrisch isoliert. Somit kann insbesondere eine mit mehreren Kanäle umfassende elektrische Verbindung zwischen dem ersten Steckverbindungsteil und dem zweiten Steckverbindungsteil hergestellt werden.

Bevorzugt bilden die Muster eine zweidimensionale Matrix, wobei ein Signalleiter mit einem ersten Kontaktelement in galvanischem Kontakt steht und drei zu dem ersten Kontaktelement benachbarte, das erste Kontaktelement umgebende zweite Kontaktelemente mit einer Abschirmung galvanisch verbunden sind. Damit kann auch ohne aufwändige Koaxialkontakte eine gut geschirmte elektrische Verbindung bereitgestellt werden.

Ferner wird eine Patientenliege mit zumindest einem zweiten Verbindungsteil vorgeschlagen. Durch eine Anordnung nahe am Patienten kann eine entsprechende Steckverbindung zum Anschluss von Lokalspulen mit kurzen Kabeln und/oder kabellosen Lokalspulen, insbesondere für Direkt-Verbindungs-Lokalspulen (engl. direct connect local coils), wie sie beispielsweise in den Druckschriften DE 102011079565 A1 und/oder US 20120126815 A1 beschrieben sind, verwendet werden. Insbesondere ermöglicht eine Integration des zumindest einen zweiten Verbindungsteils in eine Patientenliege eine verbesserte Benutzung und/oder Wartung aufgrund der vorab beschriebenen Vorteile.

An das zweite Verbindungsteil, insbesondere an das mögliche zumindest eine dritte elektrische Kontaktelement des zweiten Verbindungsteils, können Signalleitungen der Patientenliege und/oder eines Magnetresonanzgeräts angeschlossen werden.

Wie vorab bereits beschrieben weist das zweite Verbindungteil, insbesondere die erste Kontaktplatte und/oder das Gehäuse des zweiten Verbindungsteils, eine äußere Fläche auf, die in dem verbundenen Zustand der Kontaktfläche des ersten Verbindungsteils zugewandt ist. Zudem weist die Patientenliege eine Patientenliegenoberfläche aufweist, die äußere Fläche zumindest teilweise, insbesondere vollständig, umgibt. Vorzugsweise grenzt die Patientenliegenoberfläche im Wesentlichen stufenlos an die Verbindungsteiloberfläche an, d. h. der Übergang von der Patientenliegenoberfläche auf äußere Fläche des zweiten Verbindungsteils umfasst keine Stufe oder Stufen von maximal 5 mm Höhe, bevorzugt maximal 1 mm Höhe.

Insbesondere weist die Patientenliege mit dem zumindest einen zweiten Verbindungsteil eine bündige Oberfläche auf. Insbesondere sind die erste Kontaktplatte und gegebenenfalls auch ein möglicherweise sie umgebendes Gehäuse bündig zur Oberfläche des das zweite Verbindungsteil umgebenden Teils der Patientenliege angeordnet. Insbesondere befindet sich die Patientenliegenoberfläche und Verbindungsteiloberfläche auf der gleichen Ebene.

Die mögliche zumindest eine Aussparungsabdeckung ist vorzugsweise, zumindest im unverbundenen Zustand, ebenfalls bündig mit der sie umgebenden Oberfläche. Vorteilhafthafterweise wird die zumindest eine Aussparungsöffnung der zumindest einen Aussparung erst beim Verbinden des ersten Verbindungsteils mit dem zweiten Verbindungsteils geöffnet.

Eine derartige im Wesentlichen stufenlose und/oder bündige Anordnung der Oberflächen ermöglich eine besonders einfach Reinigung des zumindest einen zweiten Verbindungsteils.

Die Patientenliege kann insbesondere Elektronik umfassen, die unter dem zweiten Verbindungsteil angeordnet ist. Insbesondere kann die Elektronik gekapselt unter der ersten Kontaktplatte in die Patientenliege verbaut werden. Durch eine geschlossene Oberfläche des zweiten Verbindungsteils kann die Elektronik vor mechanischen Beschädigungen und vor dem Eindringen von Schmutz und/oder Flüssigkeiten geschützt werden.

Ferner wird eine Lokalspule mit zumindest einem ersten Verbindungsteil vorgeschlagen. Die Lokalspule kann beispielsweise zumindest ein Kabel umfassen, an dem das zumindest eine erste Verbindungsteil angeordnet ist und/oder das zumindest eine erste Verbindungsteil kann direkt in dem Gehäuse der Lokalspule angeordnet sein. Wie bereits angemerkt, kann in diesem Fall die Lokalspule auch als Direkt-Verbindungs-Lokalspulen bezeichnet werden.

Ferner wird ein Magnetresonanzgerät mit zumindest einem zweiten Verbindungsteil vorgeschlagen. Auch hier wird auf die bereits beschriebenen Vorteile verwiesen.
Weitere Vorteile, Merkmale und Einzelheiten der Erfindung ergeben sich aus den im Folgenden beschriebenen Ausführungsbeispielen sowie anhand der Zeichnungen. Einander entsprechende Teile sind in allen Figuren mit den gleichen Bezugszeichen versehen.

Es zeigen:
- Fig. 1: ein Magnetresonanzgerät mit einer Lokalspule, die über eine Steckverbindung an das Magnetresonanzgerät angeschlossen ist, in einer schematischen Darstellung,
- Fig. 2: eine Steckverbindung in einem unverbundenen Zustand

- Fig. 3: eine Steckverbindung in einem Zwischenzustand
- Fig. 4: eine Steckverbindung in einem verbundenen Zustand

- Fig. 5: eine Detaildarstellung einer Steckverbindung

- Fig. 6: eine weitere Steckverbindung in einem unverbundenen Zustand

- Fig. 7: eine weitere Steckverbindung in einem verbundenen Zustand

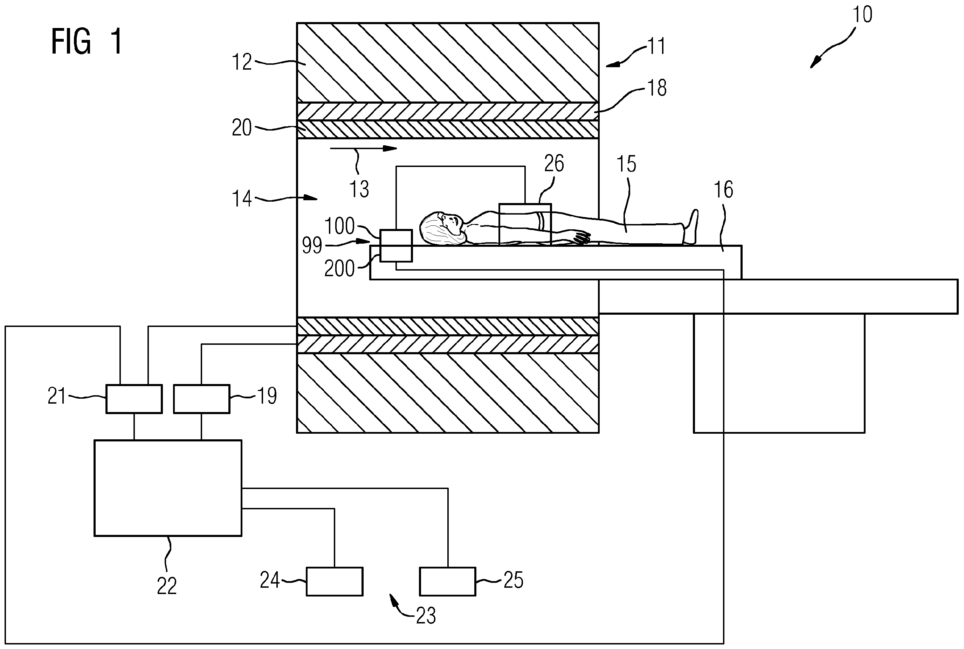

In Fig. 1 ist ein Magnetresonanzgerät 10 schematisch dargestellt. Das Magnetresonanzgerät 10 umfasst eine Magneteinheit 11, die einen supraleitenden Hauptmagneten 12 zu einem Erzeugen eines starken und insbesondere zeitlich konstanten Hauptmagnetfelds 13 aufweist. Zudem umfasst das Magnetresonanzgerät 10 einen Patientenaufnahmebereich 14 zu einer Aufnahme eines Patienten 15. Der Patientenaufnahmebereich 14 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Patientenaufnahmebereichs 14 jederzeit denkbar. Der Patient 15 kann mittels einer bewegbar ausgestalteten Patientenliege 16 in den Patientenaufnahmebereich 14 geschoben werden.

Die Magneteinheit 11 weist weiterhin eine Gradientenspuleneinheit 18 zu einer Erzeugung von Magnetfeldgradienten auf, die für eine Ortskodierung während einer Bildgebung verwendet werden. Die Gradientenspuleneinheit 18 wird mittels einer Gradientensteuereinheit 19 des Magnetresonanzgeräts 10 gesteuert. Die Magneteinheit 11 umfasst weiterhin eine Hochfrequenzantenneneinheit 20, welche im vorliegenden Ausführungsbeispiel als fest in das Magnetresonanzgerät 10 integrierte Körperspule ausgebildet ist. Die Hochfrequenzantenneneinheit 20 ist zu einer Anregung von Atomkernen, die sich in dem von dem Hauptmagneten 12 erzeugten Hauptmagnetfeld 13 einstellt, ausgelegt. Die Hochfrequenzantenneneinheit 20 wird von einer Hochfrequenzantennensteuereinheit 21 des Magnetresonanzgeräts 10 gesteuert und strahlt hochfrequente Magnetresonanzsequenzen in einen Untersuchungsraum ein, der im Wesentlichen von einem Patientenaufnahmebereich 14 des Magnetresonanzgeräts 10 gebildet ist. Die Hochfrequenzantenneneinheit 20 ist weiterhin zum Empfang von Magnetresonanzsignalen ausgebildet.

Das Magnetresonanzgerät umfasst ferner eine Lokalspule 26, die auch zum Empfang von Magnetresonanzsignalen ausgebildet ist. Die Lokalspule 26 umfasst in diesem Ausführungsbeispiel ein Kabel, an dem endseitig ein erstes Verbindungsteil 100 angeordnet ist. Das erste Verbindungsteil 100 ist hier mit einem zweiten Verbindungsteil 200 verbunden, das in der Patientenliege 16 angeordnet ist. Das erste Verbindungsteil 100 und das zweite Verbindungsteil 200 sind Teile einer Steckverbindung 99. Die zweite Steckverbindung ist mit der Hochfrequenzantennensteuereinheit 21 verbunden, so dass von der Lokalspule 26 empfangene Magnetresonanzsignale weitergeleitet werden können.

Zu einer Steuerung des Hauptmagneten 12, der Gradientensteuereinheit 19 und zur Steuerung der Hochfrequenzantennensteuereinheit 21 weist das Magnetresonanzgerät 10 eine Systemsteuereinheit 22 auf. Die Systemsteuereinheit 22 steuert zentral das Magnetresonanzgerät 10, wie beispielsweise das Durchführen einer vorbestimmten bildgebenden Gradientenechosequenz. Zudem umfasst die Systemsteuereinheit 22 eine nicht näher dargestellte Auswerteeinheit zu einer Auswertung von medizinischen Bilddaten, die während der Magnetresonanzuntersuchung erfasst werden. Des Weiteren umfasst das Magnetresonanzgerät 10 eine Benutzerschnittstelle 23, die mit der Systemsteuereinheit 22 verbunden ist. Steuerinformationen wie beispielsweise Bildgebungsparameter, sowie rekonstruierte Magnetresonanzbilder können auf einer Anzeigeeinheit 24, beispielsweise auf zumindest einem Monitor, der Benutzerschnittstelle 23 für ein medizinisches Bedienpersonal angezeigt werden. Weiterhin weist die Benutzerschnittstelle 23 eine Eingabeeinheit 25 auf, mittels der Informationen und/oder Parameter während eines Messvorgangs von dem medizinischen Bedienpersonal eingegeben werden können.

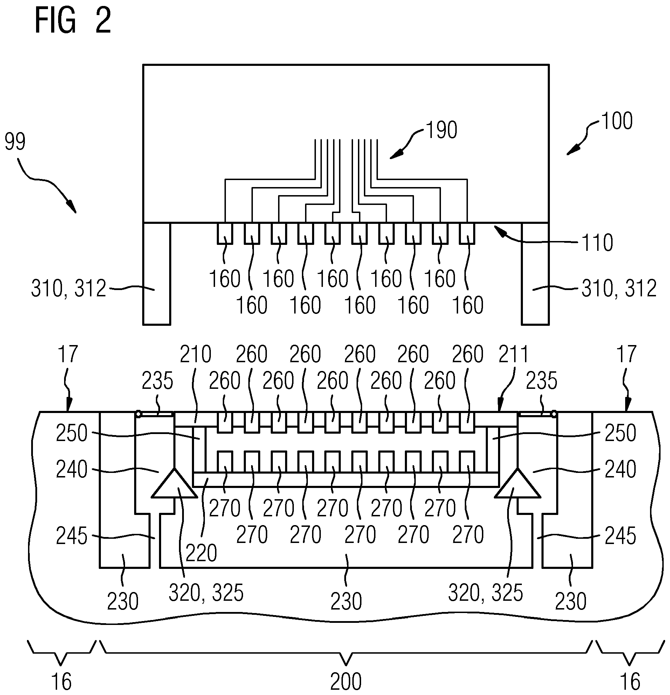

In Fig. 2 wird eine Steckverbindung 99 in einer Schnittdarstellung illustriert. Die Steckverbindung umfasst ein erstes Verbindungsteil 100 und ein zweites Verbindungsteil 200, die ausgebildet sind, lösbar miteinander verbunden zu werden, wobei Fig. 2 die Steckverbindung 99 in einem unverbundenen Zustand zeigt.

Das erste Verbindungsteil 200 umfasst eine erste Kontaktfläche, die mehrere erste elektrische Kontaktelemente 160 aufweist. Ferner wird schematisch angedeutet, dass die Kontaktelemente 160 mit elektrischen Leitungen 190 kontaktiert sind, über die elektrische Signale und/oder Energie transportiert werden können.

Das zweite Verbindungsteil 200 umfasst eine erste Kontaktplatte 210, eine zweite Kontaktplatte 220 und ein Gehäuse 230. Die zweite Kontaktplatte 220 ist relativ zum Gehäuse 230 beweglich angeordnet, was auch aus den weiteren Figuren hervorgeht. Dagegen ist die erste Kontaktplatte 210 ortsfest am Gehäuse angeordnet.

Die erste Kontaktplatte 210 umfasst mehrere zweite elektrische Kontaktelemente 260 und die zweite Kontaktplatte mehrere dritte elektrische Kontaktelemente 270. Auch die mehreren dritten elektrischen Kontakte 270 sind üblicherweise wie die mehreren ersten elektrischen Kontakte 160 mit Leitungen verbunden, auf deren Darstellung zur Erhöhung der Übersichtlichkeit verzichtet wurde.

Im unverbundenen Zustand sind die erste Kontaktplatte 210, und damit die mehreren zweiten elektrischen Kontaktelemente 260, und die zweite Kontaktplatte 220, und damit die mehreren dritten elektrischen Kontaktelemente 260, voneinander beabstandet und somit galvanisch getrennt. Das zweite Verbindungsteil 100 umfasst eine Federeinheit 250, die zwischen der zweite Kontaktplatte 220 und der ersten Kontaktplatte 210 eine abstoßende Kraft ausübt und damit die Beabstandung im unverbundenen Zustand sicherstellt. Die Federeinheit 250 kann beispielsweise eine oder mehrere Druckfedern umfassen.

Da es sich in den Fig. 2 bis 7 um Schnittdarstellungen handelt, sei darauf hingewiesen, dass es natürlich denkbar ist, dass die elektrischen Kontaktelemente 260 auch beispielsweise in einer zweidimensionale Matrix senkrecht zur Darstellungsebene angeordnet sein können. Die Steckverbindung 99 kann also in weiteren Schnittebenen weitere Kontaktelemente umfassen als nur diejenigen, die in der dargestellten Schnittebene gezeigt werden.

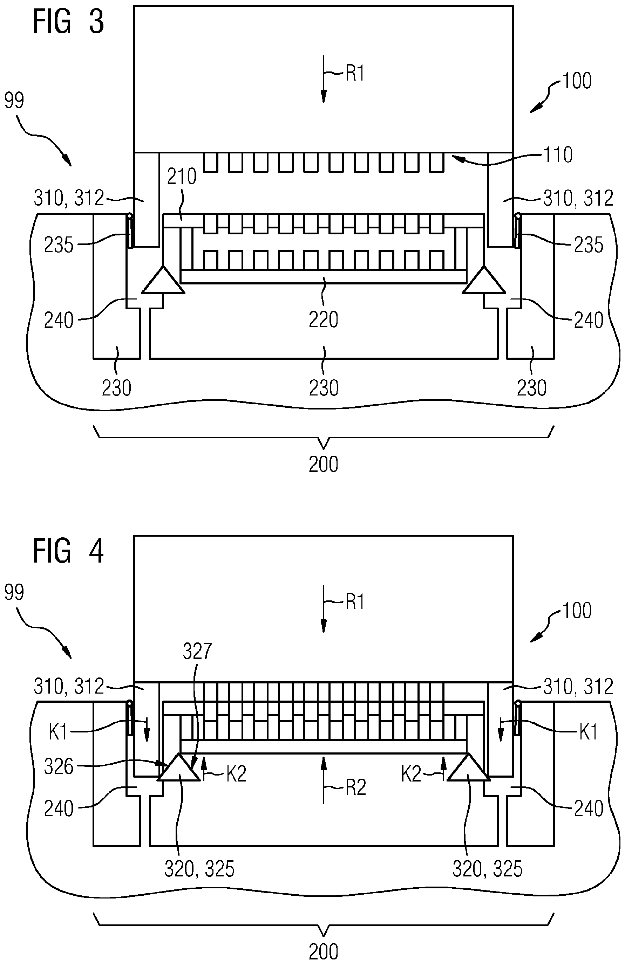

Im einem verbundenen Zustand, wie er beispielhaft in Fig. 4 dargestellt ist, berührt jeder der mehreren ersten elektrischen Kontakte 160 einen der mehreren zweiten elektrischen Kontakte 260 und wiederum jeder der zweiten elektrischen Kontakte 260 einen der mehreren dritten elektrischen Kontakte 270. Somit können in einem verbunden Zustand elektrische Signale und/oder Energie über die Steckverbindung 99 transportiert werden.

Weiter wird in Fig. 2 gezeigt, dass die erste Kontaktfläche 110, die erste Kontaktplatte 210 und die zweite Kontaktplatte umfassen jeweils einen Isolierkörper umfasst, der die Kontaktelemente voneinander trennt, um einen Kurzschluss zwischen den Kontaktelementen 160, 260, 270 zu vermeiden. Auf die Einfügung von Bezugszeichen für die Isolierkörper wurde zu Gunsten einer besseren Übersichtlichkeit der Figuren verzichtet. Insbesondere der Isolierkörper der ersten Kontaktplatte 210 kann dabei auch monolithisch mit dem Gehäuse 230 verbunden sein.

Die Steckverbind umfasst zudem eine mechanische Hebevorrichtung, die ausgebildet ist, bei einem Verbinden des ersten Verbindungsteils 100 mit dem zweiten Verbindungsteil 200 die zweite Kontaktplatte 220 in Richtung der ersten Kontaktplatte 210 zu bewegen. Die Hebevorrichtung umfasst ein erstes Hebevorrichtungsteil 310 und ein zweites Hebevorrichtungsteil 320 umfasst, wobei das erste Verbindungsteil 100 das erste Hebevorrichtungsteil 310 umfasst und das zweite Verbindungsteil 200 das zweite Hebevorrichtungsteil 320 umfasst.

Das erste Hebevorrichtungsteil 310 umfasst hier zwei vorstehende Elemente 312. Dazu passend weist das Gehäuse 230 des zweiten Verbindungsteils 200 zwei Aussparungen 240 auf, in die die vorstehenden Elemente angeordnet werden können, wie in den Fig. 3, 4, und 7 gezeigt wird. Das zweite Hebevorrichtungsteil 320 umfasst im ersten Ausführungsbeispiel zwei Hebekeile 325.

Auch hier sei wieder darauf hingewiesen, dass es sich in den Fig. 2 bis 7 um Schnittdarstellungen handelt und in weiteren Schnittebenen insbesondere weitere erste und zweite Hebevorrichtungsteile 310, 320 angeordnet sein können.

Die Aussparungen 240 werden im unverbundenen Zustand mittels Aussparungsabdeckung 235 abgedeckt, damit möglichst wenige Fremdsubstanzen, wie z. B. Schmutz, Flüssigkeiten etc., in die Aussparung eindringen können. Etwaige dennoch eindringende Fremdsubstanzen können über eine Ableitungseinheit, die hier zwei Ableitungskanäle 245 umfasst, aus dem zweiten Verbindungsteil wieder ausgeleitet werden.

Die erste Kontaktplatte 210 und das Gehäuse weisen eine äußere Fläche 211 auf, die in einem verbundenen Zustand, wie beispielhaft in Fig. 4 dargestellt, der Kontaktfläche 110 des ersten Verbindungsteil zugewandt sind. Dabei weist die äußere Fläche der ersten Kontaktplatte und des Gehäuses eine glatte und/oder in sich geschlossene Oberfläche auf. Dadurch kann das zweite Verbindungsteil 200 leicht gereinigt werden.

In Fig. 3 wird ein Zwischenzustand zwischen einem unverbundenen Zustand gemäß Fig. 2 und einem verbunden Zustand gemäß Fig. 4 dargestellt. Das erste Verbindungsteil 100 wird ausgehend vom unverbundenen Zustand relativ zum Gehäuse 230 des zweiten Verbindungsteils 200 in eine erste Verbindungsrichtung R1 bewegt, so dass sich die erste Kontaktfläche 110 an die erste Kontaktplatte 210 annähert.

Dabei dringen die vorstehenden Elemente 310, 312 in die Aussparungen 240 ein. Um das Eindringen der vorstehenden Elemente 310, 312 in die Aussparungen 240 zu ermöglichen, werden die Aussparungsabdeckungen 235 aus ihrer ursprünglichen Stellung, in der sie beispielsweise mittels einer Feder gehalten werden, weggeklappt.

Im weiteren Verlauf des Verbindens des ersten Verbindungsteils 100 mit dem zweiten Verbindungsteils 200 wechselwirkt das erste Hebevorrichtungsteil 310, hier die vorstehenden Elemente 312, mit den zweiten Hebevorrichtungsteil 320, hier die Hebekeile 325, bis ein verbundener Zustand entsteht, wie er in Fig. 4 dargestellt ist. Die Wechselwirkung bewirkt eine Bewegung der zweiten Kontaktplatte 220 relativ zum Gehäuse in eine zweite Verbindungsrichtung R2.

Beim Verbinden des ersten Verbindungsteils 100 mit dem zweiten Verbindungsteil 200 wird also das erste Verbindungsteil 100 in die erste Verbindungsrichtung R1 und die zweite Kontaktplatte 220 der zweiten Verbindungsrichtung 200 in die zweite Verbindungsrichtung R2 bewegt, wobei die erste Verbindungsrichtung und die zweite Verbindungsrichtung entgegengesetzt sind, also einen Winkel von 180° einschließen. Es sind aber auch Ausführungsformen denkbar, die nicht-parallele Bewegungen des ersten Verbindungsteils 100 und der zweiten Kontaktplatte vorsehen, d. h. der eingeschlossene Winkel liegt zwischen 90° und 180°.

Insbesondere wird beim Verbinden des ersten Verbindungsteils 100 mit dem zweiten Verbindungsteil 200 durch das erste Hebevorrichtungsteil 310 eine erste mechanische Kraft K1 auf das zweite Hebevorrichtungsteil 320 eingeleitet wird, so dass durch das zweite Hebevorrichtungsteil 320 eine zweite mechanische Kraft K2 auf die zweite Kontaktplatte 220 eingeleitet wird.

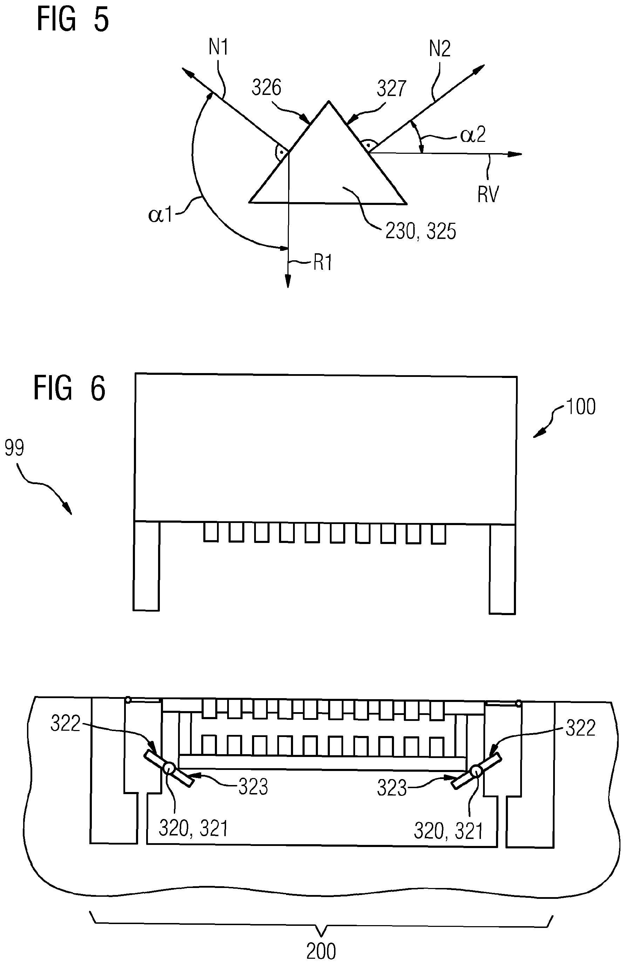

Eine detailliertere Darstellung dieses beispielhaften mechanischen Hebemechanismus unter Verwendung eines Hebekeils 320, 325 ist in Fig. 5 dargestellt. Dabei entspricht der in Fig. 5 dargestellte Hebekeil 300, 325 dem linken der beiden in den Fig. 2 bis 4 dargestellten Hebekeilen 300, 325.

Der Hebekeil weist eine erste Keilfläche 326 auf, wobei der Hebekeil derart angeordnet und/oder ausgebildet ist, dass das das erste Hebevorrichtungsteil 310, hier das vorstehende Element 312, beim Verbinden des ersten Verbindungsteils 100 mit dem zweiten Verbindungsteil 200 in eine Richtung R1 bewegt wird, die mit der Normalen N1 der ersten Keilfläche 326 einen ersten Neigungswinkel α₁ größer als 90° und kleiner also 180° einschließt. Dadurch erfährt der Hebekeil eine Kraftwirkung, die zumindest teilweise senkrecht zur Richtung R1 ausgerichtet ist. Mit Hilfe einer geeigneten Zwangsführung kann der Hebekeil somit in eine Verschiebungsrichtung RV bewegt werden, die in diesem Beispiel senkrecht zur Richtung R1 ausgerichtet ist.

Ferner weist der zumindest eine Hebekeil 325 eine zweite Keilfläche 327 auf, deren Normale N2 gegenüber einer Verschiebungsrichtung RV einen zweiten Neigungswinkel α₂ größer als 0° und kleiner als 90° einschließt. Dadurch erfährt die geeignet angeordnete zweite eine Kraftwirkung, die zumindest teilweise senkrecht zur Richtung R1 ausgerichtet ist. Mit Hilfe einer geeigneten Zwangsführung kann der Hebekeil somit in eine Verschiebungsrichtung RV bewegt werden, die in diesem Beispiel senkrecht zur Richtung R1 ausgerichtet ist.

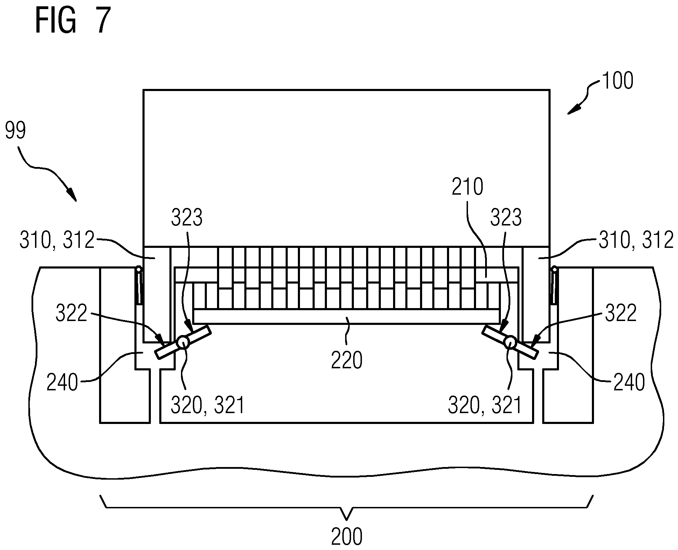

Ein weiterer möglicher Hebemechanismus ist in den Fig. 6 und 7 dargestellt. Hier umfasst das zweite Hebevorrichtungsteil 320 zwei Wippen 321.

In Fig. 6 ist ein unverbundener Zustand gezeigt, d. h. das erste Verbindungsteil 100 und das zweite Verbindungsteil 200 sind getrennt. Die Wippen weisen jeweils eine erste Wippenfläche 322 und eine zweite Wippenfläche 323 auf. Wie in Fig. 7 gezeigt wird, wird die Wippe durch das Einführen der vorstehenden Elemente 310, 312 eine mechanische Kraft auf die erste Wippenfläche 322 ausgeübt, so dass sich die Wippen 320,321 und damit auch die zweite Wippenfläche 323 drehen.

Die zweite Wippenfläche 323 ist an der zweiten Kontaktplatte angeordnet, so dass diese durch das Drehen der Wippen 320,321 in Richtung der ersten Kontaktplatte bewegt wird, bis sich die Kontaktplatten miteinander in Kontakt stehen.

Wie in den Fig. 4 und 7 angedeutet, weisen die erste Kontaktfläche und die zweite Kontaktplatte Federkontakte auf. Damit können die ersten elektrischen Kontaktelemente 260 und die dritten elektrischen Kontaktelemente 270 bei Berührung mit der ersten Kontaktplatte einfedern, so dass eine besonders zuverlässige Kontaktierung zu den zweiten elektrischen Kontaktelementen erreicht wird.

## Patentansprüche

1. Magnetresonanzgerät (10) oder eine Patientenliege (16) mit zumindest einem zweiten Verbindungsteil (200), welches ausgebildet ist, mit einem ersten Verbindungsteil (100) eine Steckverbindung (99) zum Einsatz in einem Magnetresonanzgerät einzugehen,
wobei die Steckverbindung eine elektrische Steckverbindung ist,
wobei das erste Verbindungsteil (100) und das zweite Verbindungsteil (200) ausgebildet sind, lösbar miteinander verbunden zu werden,
wobei das erste Verbindungsteil (100) eine erste Kontaktfläche (110) umfasst,
wobei das zweite Verbindungsteil (200) eine erste Kontaktplatte (210), eine zweite Kontaktplatte (220) und ein Gehäuse (230) umfasst,
wobei die zweite Kontaktplatte (220) relativ zum Gehäuse (230) beweglich angeordnet ist,
wobei in einem verbundenen Zustand die erste Kontaktplatte (210) zwischen der ersten Kontaktfläche (110) und der zweiten Kontaktplatte (220) angeordnet ist,
wobei die Steckverbindung (99) eine mechanische Hebevorrichtung umfasst, die ausgebildet ist, bei einem Verbinden des ersten Verbindungsteils (100) mit dem zweiten Verbindungsteil (200) die zweite Kontaktplatte (220) in Richtung der ersten Kontaktplatte (210) zu bewegen,
wobei die erste Kontaktfläche (110) zumindest ein erstes elektrisches Kontaktelement (160) aufweist,
wobei die erste Kontaktplatte (210) zumindest ein zweites elektrisches Kontaktelement (260) aufweist,
wobei die zweite Kontaktplatte (220) zumindest ein drittes elektrisches Kontaktelement (270) aufweist,
wobei in dem verbundenen Zustand das zumindest eine erste elektrische Kontaktelement (160) das zumindest eine zweite elektrische Kontaktelement (260) berührt und das zumindest eine zweite elektrische Kontaktelement (260) das zumindest eine dritte elektrische Kontaktelement (270) berührt.

2. Magnetresonanzgerät (10) oder eine Patientenliege (16) nach Anspruch 1,
wobei das zweite Verbindungsteil eine äußere Fläche (211) aufweist, die in dem verbundenen Zustand der Kontaktfläche (110) des ersten Verbindungsteils (100) zugewandt ist,
wobei die äußere Fläche (211) der ersten Kontaktplatte (210) und/oder des Gehäuses (230) eine glatte und/oder in sich geschlossene Oberfläche aufweist.

3. Magnetresonanzgerät (10) oder eine Patientenliege (16) nach einem der Ansprüche 1 oder 2,
wobei sich beim Verbinden des ersten Verbindungsteils (100) mit dem zweiten Verbindungsteil (200) das erste Verbindungsteil (100) in eine erste Verbindungsrichtung (R1) und die zweite Kontaktplatte (220) der zweiten Verbindungsrichtung (200) in eine zweite Verbindungsrichtung (R2) bewegt,
wobei die erste Verbindungsrichtung (R1) und die zweite Verbindungsrichtung (R2) einen Winkel von mindestens 90° und höchstens 180° einschließen.

4. Magnetresonanzgerät (10) oder eine Patientenliege (16) nach einem der vorangehenden Ansprüche,
wobei die mechanische Hebevorrichtung ausgebildet ist, bei dem Verbinden des ersten Verbindungsteils (100) mit dem zweiten Verbindungsteil (200) eine mechanische Kraft vom ersten Verbindungsteil (100) auf die zweite Kontaktplatte (220) zu übertragen.

5. Magnetresonanzgerät (10) oder eine Patientenliege (16) nach einem der vorangehenden Ansprüche,
wobei die mechanische Hebevorrichtung ein erstes Hebevorrichtungsteil (310) und ein zweites Hebevorrichtungsteil (320) umfasst,
wobei das erste Verbindungsteil (100) das erste Hebevorrichtungsteil (310) umfasst und das zweite Verbindungsteil (200) das zweite Hebevorrichtungsteil (320) umfasst,
wobei bei dem Verbinden des ersten Verbindungsteils (100) mit dem zweiten Verbindungsteil (200) durch das erste Hebevorrichtungsteil (310) eine erste mechanische Kraft (K1) auf das zweite Hebevorrichtungsteil (320) eingeleitet wird, so dass durch das zweite Hebevorrichtungsteil (320) eine zweite mechanische Kraft (K2) auf die zweite Kontaktplatte (220) eingeleitet wird.

6. Magnetresonanzgerät (10) oder eine Patientenliege (16) nach Anspruch 5,
wobei das Gehäuse (230) des zweiten Verbindungsteils (200) zumindest eine Aussparung (240) aufweist,
wobei das erste Hebevorrichtungsteil (310) zumindest ein vorstehendes Element (312) umfasst, das in der zumindest einen Aussparung (240) des Gehäuses (230) anordbar ist.

7. Magnetresonanzgerät (10) oder eine Patientenliege (16) (99) nach einem der Ansprüche 5 oder 6, wobei das zweite Hebevorrichtungsteil (320) zumindest eine Wippe (321) und/oder zumindest einen Hebekeil (325) zur Kraftumlenkung umfasst.

8. Magnetresonanzgerät (10) oder eine Patientenliege (16) nach Anspruch 7,
wobei der zumindest eine Hebekeil (325) eine erste Keilfläche (326) aufweist,
wobei der zumindest eine Hebekeil (325) derart angeordnet und/oder ausgebildet ist, dass das erste Hebevorrichtungsteil (310) beim Verbinden des ersten Verbindungsteils (100) mit dem zweiten Verbindungsteil (200) in eine Richtung (R1) bewegt wird, die mit der Normalen (N1) der ersten Keilfläche (326) einen ersten Neigungswinkel (α₁) größer als 90° und kleiner also 180° einschließt.

9. Magnetresonanzgerät (10) oder eine Patientenliege (16) nach Anspruch 8,
wobei der zumindest eine Hebekeil (325) eine zweite Keilfläche (327) aufweist, deren Normale (N2) gegenüber einer Verschiebungsrichtung (RV) einen zweiten Neigungswinkel (α₂) größer als 0° und kleiner als 90° einschließt.

10. Magnetresonanzgerät (10) oder eine Patientenliege (16) nach einem der vorangehenden Ansprüche,
wobei das zweite Verbindungsteil (200) eine Federeinheit (250) umfasst, die zwischen der zweite Kontaktplatte (220) und der ersten Kontaktplatte (210) eine abstoßende Kraft ausübt.

11. Magnetresonanzgerät (10) oder eine Patientenliege (16) nach Anspruch 10,
wobei die Federeinheit (250) zumindest eine Druckfeder umfasst.

12. Magnetresonanzgerät (10) oder eine Patientenliege (16) nach einem der vorangehenden Ansprüche,
wobei das zumindest eine erste elektrische Kontaktelement (160) und/oder das zumindest eine zweite elektrische Kontaktelement (260) und/oder das zumindest eine dritte elektrische Kontaktelement (270) zumindest einen Federkontakt aufweisen.

13. Patientenliege (16) nach einem der vorangehenden Ansprüche,
wobei das zweite Verbindungsteil eine äußere Fläche (211) aufweist, die in dem verbundenen Zustand der Kontaktfläche (110) des ersten Verbindungsteils (100) zugewandt ist,
wobei die Patientenliege (16) eine Patientenliegenoberfläche (17) aufweist, die die äußere Fläche (211) zumindest teilweise umgibt,
wobei die Patientenliegenoberfläche (17) im Wesentlichen stufenlos an die äußere Fläche (211) angrenzt.

14. Lokalspule (26) mit zumindest einem ersten Verbindungsteil (100), welches ausgebildet ist, mit einem zweiten Verbindungsteil (200) eine Steckverbindung (99) einzugehen, wobei die Steckverbindung eine elektrische Steckverbindung ist,
wobei das erste Verbindungsteil (100) und das zweite Verbindungsteil (200) ausgebildet sind, lösbar miteinander verbunden zu werden,
wobei das erste Verbindungsteil (100) eine erste Kontaktfläche (110) umfasst,
wobei das zweite Verbindungsteil (200) eine erste Kontaktplatte (210), eine zweite Kontaktplatte (220) und ein Gehäuse (230) umfasst,
wobei die zweite Kontaktplatte (220) relativ zum Gehäuse (230) beweglich angeordnet ist,
wobei in einem verbundenen Zustand die erste Kontaktplatte (210) zwischen der ersten Kontaktfläche (110) und der zweiten Kontaktplatte (220) angeordnet ist,
wobei die Steckverbindung (99) eine mechanische Hebevorrichtung umfasst, die ausgebildet ist, bei einem Verbinden des ersten Verbindungsteils (100) mit dem zweiten Verbindungsteil (200) die zweite Kontaktplatte (220) in Richtung der ersten Kontaktplatte (210) zu bewegen,
wobei die erste Kontaktfläche (110) zumindest ein erstes elektrisches Kontaktelement (160) aufweist,
wobei die erste Kontaktplatte (210) zumindest ein zweites elektrisches Kontaktelement (260) aufweist,
wobei die zweite Kontaktplatte (220) zumindest ein drittes elektrisches Kontaktelement (270) aufweist,
wobei in dem verbundenen Zustand das zumindest eine erste elektrische Kontaktelement (160) das zumindest eine zweite elektrische Kontaktelement (260) berührt und das zumindest eine zweite elektrische Kontaktelement (260) das zumindest eine dritte elektrische Kontaktelement (270) berührt.

## Claims

1. Magnetic resonance device (10) or a patient couch (16) having at least one second connecting part (200), which is embodied, with a first connecting part (100), to coalesce a plug connector (99) for use in a magnetic resonance device, wherein the plug connector is an electrical plug connector, wherein the first
connecting part (100) and the second connecting part (200) are embodied to be connected detachably to one another,
wherein the first connecting part (100) comprises a first contact surface (110),
wherein the second connecting part (200) comprises a first contact plate (210), a second contact plate (220) and a housing (230),
wherein the second contact plate (220) is arranged so that it can be moved relative to the housing (230),
wherein, in a connected state, the first contact plate (210) is arranged between the first contact surface (110) and the second contact plate (220),
wherein the plug connector (99) comprises a mechanical lifting apparatus, which is embodied, when the first connecting part (100) is connected to the second connecting part (200), to move the second contact plate (220) in the direction of the first contact plate (210),
wherein the first contact surface (110) has at least one first electrical contact element (160),
wherein the first contact plate (210) has at least one second electrical contact element (260),
wherein the second contact plate (220) has at least one third electrical contact element (270),
wherein in the connected state the at least one first electrical contact element (160) makes contact with the at least one second electrical contact element (260) and the at least one second electrical contact element (260) makes contact with the at least one third electrical contact element (270) .

2. Magnetic resonance device (10) or a patient couch (16) according to claim 1,
wherein the second connecting part has an outer surface (211), which in the connected state faces towards the contact surface (110 of the first connecting part (100),
wherein the outer surface (211) of the first contact plate (210) and/or of the housing (230) has a smooth and/or coherent surface.

3. Magnetic resonance device (10) or a patient couch (16) according to one of claims 1 or 2,
wherein, on connection of the first connecting part (100) to the second connecting part (200) the first connecting part (100) moves in a first connecting direction (R1) and the second contact plate (220) of the second connecting direction (200) moves in a second connecting direction (R2),
wherein the first connecting direction (R1) and the second connecting direction (R2) enclose an angle of at least 90° and at most 180°.

4. Magnetic resonance device (10) or a patient couch (16) according to one of the preceding claims,
wherein the mechanical lifting apparatus is embodied, when the first connecting part (100) is being connected to the second connecting part (200), to transmit a mechanical force from the first connecting part (100) to the second contact plate (220).

5. Magnetic resonance device (10) or a patient couch (16) according to one of the preceding claims,
wherein the mechanical lifting apparatus comprises a first lifting apparatus part (310) and a second lifting apparatus part (320),
wherein the first connecting part (100) comprises the first lifting apparatus part (310) and the second connecting part (200) comprises the second lifting apparatus part (320), wherein, when the first connecting part (100) is being connected to the second connecting part (200), a first mechanical force (K1) is introduced into the second lifting apparatus part (320) by the first lifting apparatus part (310), so that a second mechanical force (K2) is introduced into the second contact plate (220) by the second lifting apparatus part (320).

6. Magnetic resonance device (10) or a patient couch (16) according to claim 5,
wherein the housing (230) of the second connecting part (200) has at least one recess (240),
wherein the first lifting apparatus part (310) comprises at least one projecting element (312), which is able to be arranged in the at least one recess (240) of the housing (230) .

7. Magnetic resonance device (10) or a patient couch (16) according to one of claims 5 or 6,
wherein the second lifting apparatus part (320) comprises at least one rocker (321) and/or at least one lifting wedge (325) for force deflection.

8. Magnetic resonance device (10) or a patient couch (16) according to claim 7,
wherein the at least one lifting wedge (325) has a first wedge surface (326),
wherein the at least one lifting wedge (325) is arranged and/or is embodied so that the first lifting apparatus part (310) is moved in a direction (R1) when the first connecting part (100) is being connected to the second connecting part (200), which encloses a first angle of inclination (α₁) greater than 90° and less than 180° with the normal (N1) of the first wedge surface (326).

9. Magnetic resonance device (10) or a patient couch (16) according to claim 8,
wherein the at least one lifting wedge (325) has a second wedge surface (327), of which the normal (N2) encloses a second angle of inclination (α₂) greater than 0° and less than 90° in relation to a direction of displacement (RV).

10. Magnetic resonance device (10) or a patient couch (16) according to one of the preceding claims,
wherein the second connecting part (200) comprises a spring unit (250), which exerts a repelling force between the second contact plate (220) and the first contact plate (210).

11. Magnetic resonance device (10) or a patient couch (16) according to claim 10,
wherein the spring unit (250) comprises at least one compression spring.

12. Magnetic resonance device (10) or a patient couch (16) according to one of the preceding claims,
wherein the at least one first electrical contact element (160) and/or the at least one second electrical contact element (260) and/or the at least one third electrical contact element (270) have at least one spring contact.

13. Patient couch (16) according to one of the preceding claims,
wherein the second connecting part has an outer surface (211), which, in the connected state, faces towards the contact surface (110) of the first connecting part (100),
wherein the patient couch (16) has a patient support surface (17), which at least partly encloses the outer surface (211), wherein the patient support surface (17) essentially adjoins the outer surface (211) steplessly.

14. Local coil (26) with at least one first connecting part (100) which is embodied to coalesce a plug connector (99) with a second connecting part (200), wherein the plug connector is an electrical plug connector,
wherein the first connecting part (100) and the second connecting part (200) are embodied to be connected detachably to one another,
wherein the first connecting part (100) comprises a first contact surface (110),
wherein the second connecting part (200) comprises a first contact plate (210), a second contact plate (220) and a housing (230),
wherein the second contact plate (220) is arranged so that it can be moved relative to the housing (230),
wherein, in a connected state, the first contact plate (210) is arranged between the first contact surface (110) and the second contact plate (220),
wherein the plug connector (99) comprises a mechanical lifting apparatus, which is embodied, when the first connecting part (100) is connected to the second connecting part (200), to
move the second contact plate (220) in the direction of the first contact plate (210),
wherein the first contact surface (110) has at least one first electrical contact element (160),
wherein the first contact plate (210) has at least one second electrical contact element (260),
wherein the second contact plate (220) has at least one third electrical contact element (270),
wherein in the connected state the at least one first electrical contact element (160) makes contact with the at least one second electrical contact element (260) and the at least one second electrical contact element (260) makes contact with the at least one third electrical contact element (270) .

## Revendications

1. Appareil (10) à résonance magnétique ou couchette (16) de patient, comprenant au moins une deuxième partie (200) de liaison , qui est constituée pour entrer en une liaison (99) par enfichage avec une première partie (100) de liaison pour l'insertion dans un appareil à résonance magnétique,
dans lequel la liaison par enfichage est une liaison par enfichage électrique,
dans lequel la première partie (100) de liaison et la deuxième partie (200) de liaison sont constituées de manière à pouvoir être reliées entre elles de façon détachable,
dans lequel la première partie (100) de liaison comprend une première surface (110) de contact,
dans lequel la deuxième partie (200) de liaison comprend une première plaque (210) de contact, une deuxième plaque (220) de contact et un boîtier (230),
dans lequel la deuxième plaque (220) de contact est montée mobile par rapport au boîtier (230),
dans lequel, à l'état relié, la première plaque (210) de contact est disposée entre la première surface (110) de contact et la deuxième plaque (220) de contact,
dans lequel la liaison (99) par enfichage comprend un système mécanique de levage, qui est constitué pour, lors d'une liaison de la première partie (100) de liaison à la deuxième partie (200) de liaison, déplacer la deuxième plaque (220) de contact en direction de la première plaque (210) de contact,
dans lequel la première surface (110) de contact a au moins un premier élément (160) de contact électrique,
dans lequel la première plaque (210) de contact a au moins un deuxième élément (260) de contact électrique,
dans lequel la deuxième plaque (220) de contact a au moins un troisième élément (270) de contact électrique,
dans lequel, à l'état relié, le au moins un premier élément (160) de contact électrique touche le au moins un deuxième élément (260) de contact électrique et le au moins un deuxième élément (260) de contact électrique touche le au moins un troisième élément (270) de contact électrique.

2. Appareil (10) à résonance magnétique ou couchette (16) de patient suivant la revendication 1,
dans lequel la deuxième partie de liaison a une surface (211) extérieure, qui, à l'état relié, est tournée vers la surface (110) de contact de la première partie (100) de liaison,
dans lequel la surface (211) extérieure de la première plaque (210) de contact et/ou du boîtier (230) a une surface lisse et/ou en soi unie.

3. Appareil (10) à résonance magnétique ou couchette (16) de patient suivant l'une des revendications 1 ou 2,
dans lequel, lors de la liaison de la première partie (100) de liaison à la deuxième partie (200) de liaison, la première partie (100) de liaison se déplace dans une première direction (R1) de liaison et la deuxième plaque (220) de contact de la deuxième partie (200) de liaison dans une deuxième direction (R2) de liaison,
la première direction (R1) de liaison et la deuxième direction (R2) de liaison faisant un angle d'au moins 90° et d'au plus 180°.

4. Appareil (10) à résonance magnétique ou couchette (16) de patient suivant l'une des revendications précédentes,
dans lequel le système mécanique de levage est constitué de manière à, lors de la liaison de la première partie (100) de liaison à la deuxième partie (200) de liaison, transmettre une force mécanique de la première partie (100) de liaison à la deuxième plaque (220) de contact.

5. Appareil (10) à résonance magnétique ou couchette (16) de patient suivant l'une des revendications précédentes, dans lequel le système mécanique de levage comprend une première partie (310) de système de levage et une deuxième partie (320) de système de levage,
dans lequel la première partie (100) de liaison comprend la première partie (310) de système de levage et la deuxième partie (200) de liaison comprend la deuxième partie (320) de système de levage,
dans lequel, lors de la liaison de la première partie (100) de liaison à la deuxième partie (200) de liaison, il est appliqué, par la première partie (310) du système de levage, une première force (K1) mécanique à la deuxième partie (320) du système de levage, de manière à appliquer, par la deuxième partie (320) du système de levage, une deuxième force (K2) mécanique à la deuxième plaque (220) de contact.

6. Appareil (10) à résonance magnétique ou couchette (16) de patient suivant la revendication 5,
dans lequel le boîtier (230) de la deuxième partie (200) de liaison a au moins un évidement (240),
dans lequel la première partie (310) du système de levage comprend au moins un élément (312) en saillie, qui peut être mis dans le au moins un évidement (240) du boîtier (230).

7. Appareil (10) à résonance magnétique ou couchette (16) de patient suivant l'une des revendications 5 ou 6,
dans lequel la deuxième partie (320) du système de levage comprend au moins une bascule (321) et/ou au moins un coin (325) de levage de déviation de force.

8. Appareil (10) à résonance magnétique ou couchette (16) de patient suivant la revendication 7,
dans lequel le au moins un coin (325) de levage a une première surface (326) de coin,
dans lequel le au moins un coin (325) de levage est disposé et/ou constitué de manière à déplacer la première partie (310) du système de levage lors de la liaison de la première partie (100) de liaison à la deuxième partie (200) de liaison dans une direction (R1), qui fait, avec la normale (N1) à la première surface (326) de coin, un premier angle (α₁) d'inclinaison plus grand que 90° et plus petit aussi que 180°.

9. Appareil (10) à résonance magnétique ou couchette (16) de patient suivant la revendication 8,
dans lequel le au moins un coin (325) de levage a une deuxième surface (327) de coin, dont la normale (N2) fait, avec une direction (RV) de déplacement, un deuxième angle (α₂) d'inclinaison plus grand que 0° et plus petit que 90°.

10. Appareil (10) à résonance magnétique ou couchette (16) de patient suivant l'une des revendications précédentes,
dans lequel la deuxième partie (200) de liaison comprend une unité (250) de ressort, qui applique une force de répulsion entre la deuxième plaque (220) de contact et la première plaque (210) de contact.

11. Appareil (10) à résonance magnétique ou couchette (16) de patient suivant la revendication 10,
dans lequel l'unité (250) de ressort comprend un ressort de compression.

12. Appareil (10) à résonance magnétique ou couchette (16) de patient suivant l'une des revendications précédentes,
dans lequel le au moins un premier élément (160) de contact électrique et/ou le au moins un deuxième élément (260) de contact électrique et/ou le au moins un troisième élément (270) de contact électrique ont au moins un contact à ressort.

13. Couchette (16) de patient suivant l'une des revendications précédentes,
dans lequel la deuxième partie de liaison a une surface (211) extérieure, qui, à l'état relié, est tournée vers la surface (110) de contact de la première partie (100) de liaison,
dans lequel la couchette (16) de patient a une surface où se couche le patient, qui entoure au moins en partie la surface (211) extérieure,
la surface (17) où se couche le patient étant voisine sensiblement d'une manière continue de la surface (211) extérieure.

14. Bobine (26) locale, ayant au moins une première partie (100) de liaison, qui est constituée pour entrer en une liaison (99) par enfichage avec une deuxième partie (200) de liaison, la liaison par enfichage étant une liaison par enfichage électrique,
dans laquelle la première partie (100) de liaison et la deuxième partie (200) de liaison sont constituées pour être reliées entre elles de manière détachable,
dans laquelle la première partie (100) de liaison comprend une première surface (110) de contact,
dans laquelle la deuxième partie (200) de liaison comprend une première plaque (210) de contact, une deuxième plaque (220) de contact et un boîtier (230),
dans laquelle la deuxième plaque (220) de contact est montée mobile par rapport au boîtier (230),
dans laquelle, à l'état relié, la première plaque (210) de contact est disposée entre la première surface (110) de contact et la deuxième plaque (220) de contact,
dans laquelle la liaison (99) par enfichage comprend un système mécanique de levage, qui est constitué pour, lors d'une liaison de la première partie (100) de liaison à la deuxième partie (200) de liaison, déplacer la deuxième plaque (220) de contact en direction de la première plaque (210) de contact,
dans laquelle la première surface (110) de contact a au moins un premier élément (160) de contact électrique,
dans laquelle la première plaque (210) de contact a au moins un deuxième élément (260) de contact électrique,
dans lequel la deuxième plaque (220) de contact a au moins un troisième élément (270) de contact électrique,
dans laquelle, à l'état relié, le au moins un premier élément (160) de contact électrique touche le au moins un deuxième élément (260) de contact électrique et le au moins un deuxième élément (260) de contact électrique touche le au moins un troisième élément (270) de contact électrique.
